# EUROPEAN PATENT APPLICATION

(11) **EP 4 541 199 A1**
(43) Date of publication of application: **23.04.2025**
(21) Application number: 24207301.3
(22) Date of filing: 17.10.2024
(51) Int. Cl.: A23B 7/152, G01N 33/02, G05B 13/02, G06Q 10/08, G06N 3/00, G06N 20/00

(54) **SYSTEMS AND METHODS FOR MONITORING RIPENING OF PERISHABLE ITEMS**

(30) Priority: 17.10.2023 US 202363590838 P
(71) Applicant: Carrier Corporation, Palm Beach Gardens, FL 33418 (US)
(72) Inventor: POSTGATE, Farley, Syracuse, 13221 (US); VELTMAN, Rob, 3087 BM Rotterdam (NL)
(74) Representative: Dehns

(57) **Abstract**

Embodiments of the invention describe systems (110) and methods (400) for monitoring ripening of perishable items (104) stored within a container (102). The method (400) comprises measuring (402) one or more environmental parameters within the container (102) at pre-determined time intervals. Further, the method (400) comprises determining (404) a respiration rate associated with each of the perishable items (104) based on the measured one or more environmental parameters. Further, the method (400) comprises determining (406), based on the determined respiration rate and a ripening database (204a), an initiation time for initiating ripening of the perishable items (104). Furthermore, the method (400) comprises triggering (408) the ripening of the perishable items (104) at the initiation time.

## Description

This application claims the benefit of U.S. Provisional Patent Application No. 63/590,838 filed on October 17, 2023, which is incorporated by reference herein in its entirety.

The invention generally relates to controlled environments, and more particularly relates to systems and methods for monitoring ripening of perishable items present in containers.

Generally, a perishable cargo is transported to various places in transport containers. In particular, the perishable cargo may be kept in the transport container at predetermined conditions and then transported from one place to another. The predetermined conditions within the transport container may be set prior to the transport of the perishable cargo.

Perishable cargo often includes consumable items such as fruits and vegetables. The perishable cargo may be harvested in numerous unknown conditions, and it becomes difficult to set a common controlled environment within the transport container for the perishable cargo. With the variations in characteristics within a set of perishable cargos to be transported, the common controlled environment with the predetermined conditions within the transport container may not be suitable for the entire set of perishable cargos. For instance, some particular values of predetermined conditions may be suitable for a first set of perishable cargos, however, the same particular values of the perishable cargo may not be suitable for a second set of perishable cargo.

Thus, a condition of the perishable cargo prior to transportation may not always be a known parameter. With a lack of information regarding the condition of the perishable cargo, it becomes difficult to track the condition of the perishable cargo during the transportation. Moreover, it becomes difficult to control the conditions of the perishable cargo so as to achieve a desired result. For instance, a customer may require the perishable cargo to be ripe upon reaching the destination, however, with unknown condition and characteristics of the perishable cargo, there is a challenge in setting up the controlled environment within the transport container as well as track the characteristics of the perishable cargo during the transportation.

Therefore, it would be advantageous to provide a solution that can overcome the above-discussed problems.

This summary is provided to introduce a selection of concepts, in a simplified format, that are further described in the detailed description of the disclosure. This summary is neither intended to identify key or essential inventive concepts of the invention and nor is it intended for determining the scope of the invention, which is as set out in the appended claims.

According to a first aspect of the invention there is provided a method for monitoring ripening of perishable items stored within a container. The method comprises measuring one or more environmental parameters within the container at pre-determined time intervals. Further, the method comprises determining a respiration rate associated with each of the perishable items based on the measured one or more environmental parameters. Further, the method comprises determining, based on the determined respiration rate and a ripening database, an initiation time for initiating ripening of the perishable items. Furthermore, the method comprises triggering the ripening of the perishable items at the initiation time.

Optionally, the method further comprises receiving one or more additional inputs indicative of a target time for the ripening of the perishable items. Furthermore, the method optionally comprises determining the initiation time based on the received one or more additional inputs.

Optionally, the method further comprises continue monitoring the one or more environmental parameters within the container after triggering the ripening of the perishable items. Furthermore, the method optionally comprises adjusting the one or more environmental parameters to complete the ripening of the perishable items at the target time. Adjusting the one or more environmental parameters causes a change in the respiration rate associated with each of the perishable items.

Optionally, the one or more environmental parameters include CO₂, O₂, temperature, ethylene, and humidity.

Optionally, the method further comprises storing the measured one or more environmental parameters in the ripening database.

Optionally, the method further comprises detecting activation of a purge mode with respect to the container. Furthermore, the method optionally comprises initiating monitoring of the one or more environmental parameters in response to detecting activation of the purge mode.

Optionally, the method further comprises determining whether the respiration rate is greater than a pre-determined threshold. Furthermore, the method optionally comprises upon a determination that the respiration rate is greater than a pre-determined threshold, triggering an alarm associated with the container.

According to a second aspect of the invention there is provided a system to monitor ripening of perishable items stored within a container. The system comprises a memory and one or more processors communicatively coupled to the memory. The one or more processors are configured to measure one or more environmental parameters within the container at pre-determined time intervals. Further, the one or more processors are configured to determine a respiration rate associated with each of the perishable items based on the measured one or more environmental parameters. Further, the one or more processors are configured to determine, based on the determined respiration rate and a ripening database, an initiation time for initiating ripening of the perishable items. Furthermore, the one or more processors are configured to trigger the ripening of the perishable items at the initiation time.

Optionally, the one or more processors are further configured to receive one or more additional inputs indicative of a target time for the ripening of the perishable items. Further, the one or more processors are optionally configured to determine the initiation time based on the received one or more additional inputs.

Optionally, the one or more processors are further configured to continuously monitor the one or more environmental parameters within the container after triggering the ripening of the perishable items. Further, the one or more processors are optionally configured to adjust the one or more environmental parameters to complete the ripening of the perishable items at the target time. Adjustment of the one or more environmental parameters causes a change in the respiration rate associated with each of the perishable items.

Optionally, the one or more environmental parameters include CO₂, O₂, temperature, ethylene and humidity.

Optionally, the one or more processors are further configured to store the measured one or more environmental parameters in the ripening database.

Optionally, the one or more processors are configured to detect activation of a purge mode with respect to the container. Further, the one or more processors are optionally configured to initiate monitoring of the one or more environmental parameters in response to detecting activation of the purge mode.

Optionally, the one or more processors are configured to determine whether the respiration rate is greater than a pre-determined threshold. Further, the one or more processors are optionally configured to, upon a determination that the respiration rate is greater than a pre-determined threshold, trigger an alarm associated with the container.

To further clarify the advantages and features of the methods, systems, and apparatuses, a more particular description of the methods, systems, and apparatuses will be rendered by reference to specific embodiments thereof, which are illustrated in the appended drawings. It is appreciated that these drawings depict only typical embodiments of the invention and are therefore not to be considered limiting of its scope. The invention will be described and explained with additional specificity and detail with the accompanying drawings.

These and other features, aspects, and advantages of the invention will become better understood when the following detailed description is read with reference to the accompanying drawings in which like characters represent like parts throughout the drawings. Certain exemplary embodiments will now be described in greater detail by way of example only and with reference to the accompanying drawings in which:
**Figure 1** illustrates an environment for monitoring ripening of perishable items stored within a container;
**Figure 2** illustrates a schematic block diagram of a system for monitoring ripening of the perishable items stored within the container;
**Figure 3a** illustrates the container associated with a positive pressure in a purge mode thereof;
**Figure 3b** illustrates an exemplary graphical representation of the measured values of one or more environmental parameters over a period of time; and
**Figure 4** illustrates a process flow depicting a method for monitoring ripening of the perishable items stored within the container.

Further, skilled artisans will appreciate that elements in the drawings are illustrated for simplicity and may not have necessarily been drawn to scale. For example, the flow charts illustrate the method in terms of the most prominent steps involved to help to improve understanding of aspects of the invention. Furthermore, in terms of the construction of the device, one or more components of the device may have been represented in the drawings by conventional symbols, and the drawings may show only those specific details that are pertinent to understanding the embodiments of the invention so as not to obscure the drawings with details that will be readily apparent to those of ordinary skill in the art having the benefit of the description herein.

For the purpose of promoting an understanding of the principles of the invention, reference will now be made to the various embodiments and specific language will be used to describe the same. It will nevertheless be understood that no limitation of the scope of the invention is thereby intended, such alterations and further modifications in the illustrated system, and such further applications of the principles of the invention as illustrated therein being contemplated as would normally occur to one skilled in the art to which the invention relates.

It will be understood by those skilled in the art that the foregoing general description and the following detailed description are explanatory of the invention and are not intended to be restrictive thereof.

Reference throughout this specification to "an aspect", "another aspect" or similar language means that a particular feature, structure, or characteristic described in connection with the embodiment is included in at least one embodiment of the invention. Thus, appearances of the phrase "in an embodiment", "in another embodiment", "some embodiments", "one or more embodiments" and similar language throughout this specification may but do not necessarily, all refer to the same embodiment.

The terms "comprises", "comprising", or any other variations thereof, are intended to cover a non-exclusive inclusion, such that a process or method that comprises a list of steps does not include only those steps but may include other steps not expressly listed or inherent to such process or method. Similarly, one or more devices or sub-systems or elements or structures or components proceeded by "comprises... a" does not, without more constraints, preclude the existence of other devices or other sub-systems or other elements or other structures or other components or additional devices or additional sub-systems or additional elements or additional structures or additional components.

In addition to overcoming the challenges related to monitoring perishable items, the disclosure provides for systems and methods that allow effective monitoring of ripening and respiration rates of perishable items which are to be transported from one location to another location. The conditions of the perishable items may be indicated prior to completion of the transportation. Further, the on-board parameters may be adjusted in order to meet a customer's request for desired ripening of the perishable items.

Embodiments of the invention will be described below in detail with reference to the accompanying drawings.

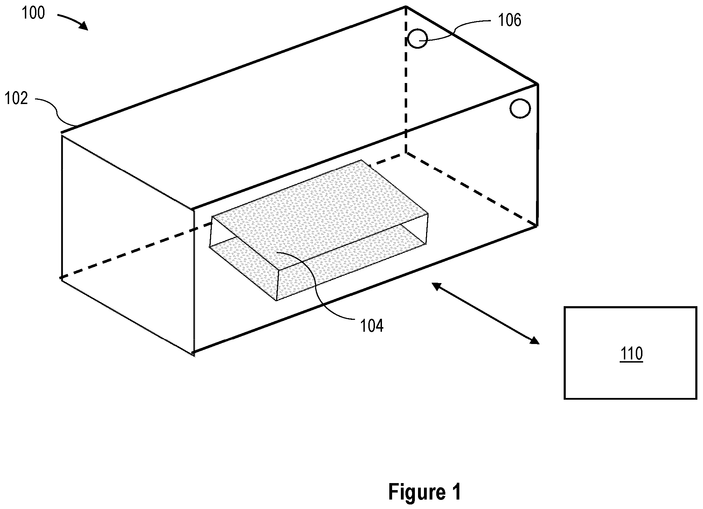

**Figure 1** illustrates an environment 100 for monitoring ripening of perishable items stored within a container 102, in accordance with one or more embodiments of the invention. The environment 100 may include the container 102 configured to store perishable items 104 there-within. The container 102 may acts as a storage enclosure for storing the perishable items 104 in controlled environment as well as a transport container for transporting the perishable items 104 from one location to another location. In an embodiment, the container 102 may be a marine container adapted to be transported by a marine vessel over water.

In an embodiment, the perishable items 104 may include fruits and/or vegetables, such as, banana, avocado, apples, melon, apricot, tomatoes, and the like. In some embodiments, the perishable items 104 may include flowers and plants.

In an embodiment, each item from among the perishable items 104 is associated with a respiration rate. The respiration rate may refer to the anaerobic respiration by the perishable items 104.

In an embodiment, the container 102 may comprise one or more sensors 106 configured to measure environmental parameters within the container 102. The one or more sensors 106 may be installed within the container 102. In some embodiments, the one or more sensors 106 may be sensors disposed independently at respective locations within the container 102. In some embodiments, the one or more sensors 106 may be integrated with a common detection unit disposed within the container 102.

By virtue of the one or more sensors 106, corresponding one or more environmental parameters may be measured within the container 102. The one or more environmental parameters may include, as non-limiting examples, carbon dioxide (CO₂), oxygen (O₂), temperature, ethylene, humidity, and the like.

The one or more sensors 106 may include corresponding sensors to measure the one or more environmental parameters within the container 102. The one or more sensors 106 may include a temperature sensor, a humidity sensor, an O₂ sensor, a CO₂ sensor, an ethylene sensor, and the like. Based on the corresponding environmental parameters sensed by the one or more sensors 106, the ripening of the perishable items may be monitored, as will be detailed further below. A person skilled in the art would understand that the details explained with respect to one container 102 may be equally applicable for multiple other containers for storing and/or transporting perishable items 104, without deviating from the scope of the invention.

In an embodiment, the container 102 comprising the perishable items 104 may be transported from one location to another location by means of a transport vehicle (not shown). The transport vehicle may include, for instance, a ship, a truck, a trailer, and the like.

The environment 100 may further include a system 110 for monitoring the ripening of the perishable items 104 within the container 102. In some embodiments, the system 110 may be communicatively coupled with the one or more sensors 106. In some embodiments, the system 110 may be communicatively coupled with the one or more sensors 106 over a communication network (not shown). In some embodiments, the system 110 may be configured to monitor the ripening of the perishable items 104 based on the readings from the one or more sensors 106.

In some embodiments, the system 100 may be configured to measure a respiratory quotient (RQ) associated with the perishable items 104. The RQ of the perishable items 104 is defined by a ratio of produced CO₂ and absorbed O₂ by corresponding perishable items 104. Based on the monitored RQ, changes in the oxygen content and carbon dioxide content can be determined. The system 100 may be configured to identify rate of change of respiration rates of the perishable items based on changes in the monitored RQ. The monitored RQ may thus facilitate predicting ripening of the perishable items. That is, the system 100 may determine an optimal time for the ripening of the perishable items 104 to begin based on the monitored RQ.

In some embodiments, the system 110 may be a server-based device that is remote to the container 102. In some embodiments, the system 110 may be a cloud-based device. In some embodiments, the system 110 may be provided within the container 102. For instance, the system 110 may be integrated with a control unit within the container 102 and the functionalities of the system 110 may be provided through the control unit. In some embodiments, one or more components of the system 110 may be provided on cloud while one or more components of the system 110 may be provided on a server.

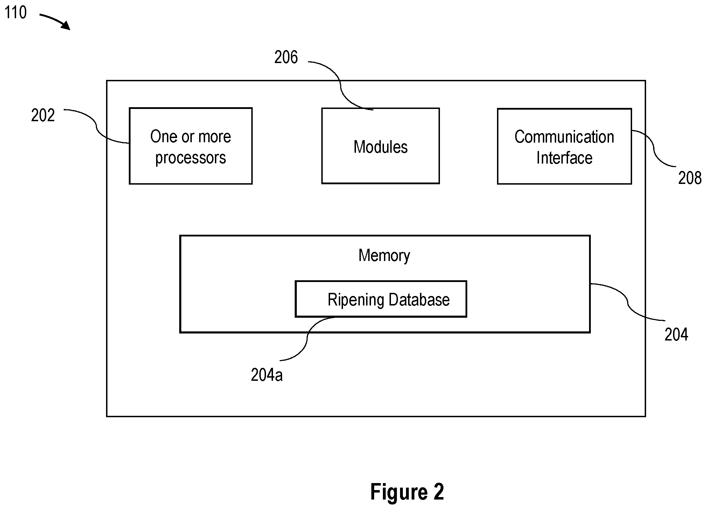

**Figure 2** illustrates a schematic block diagram of the system 110 for monitoring ripening of perishable items 104 stored within the container 102, in accordance with one or more embodiments of the invention.

In one or more embodiments, the system 110 may comprise one or more processors 202, a memory 204, one or more modules 206, and a communication interface 208.

The one or more processors 202 may be configured to communicate with the memory 204 to store sensor readings generated by the one or more sensors 106. In some embodiments, the memory 204 may comprise ripening database 204a for storing the sensor readings generated by the one or more sensors 106. The sensor readings may include real-time sensor readings as well as historic sensor readings.

In one or more embodiments, the one or more processors 202 may be one or more microprocessor(s) or microcontroller(s). The one or more processors 202 may include one or a plurality of processors, may include one or more general-purpose processors, such as a central processing unit (CPU), an application processor (AP), or the like, a graphics-only processing unit such as a graphics processing unit (GPU), a visual processing unit (VPU), and/or an Artificial intelligence (Al) dedicated processor such as a neural processing unit (NPU).

In some embodiments, the memory 204 may store data and instructions executable by the processor(s) 202 to perform the method steps for monitoring ripening of the perishable items 104, as discussed herein throughout the disclosure. The memory 204 may further include, but is not limited to, a non-transitory computer-readable storage media such as various types of volatile and non-volatile storage media, including but not limited to, random access memory, read-only memory, programmable read-only memory, electrically programmable read-only memory, electrically erasable read-only memory, flash memory, magnetic tape or disk, optical media and the like. Further, the non-transitory computer-readable storage media of memory 204 may include executable instructions in a form of the modules 206 and a unit to store data. The modules 206 may include a set of instructions that may be executed to cause the one or more processors 202 to perform any one or more of the methods for monitoring ripening of the perishable items 104, as disclosed herein throughout the disclosure. Specifically, the one or more modules 206 may be configured to perform the steps of the invention using the data stored in the unit of the memory 204 for monitoring ripening of the perishable items 104. In another embodiment, the modules 206 may be one or more hardware units that may be outside the memory 204. In one embodiment, the memory 204 may communicate via a bus within the processor(s) 202.

In one or more embodiments, the communication interface 208 may include a transmitter and a receiver configured to communicate with the one or more sensors 106, via the communication network. In some embodiments, the communication via the communication network may be based on a wireless communication protocol. The communication interface 208 may be configured for communicating internally between internal hardware components and with external devices, e.g., the one or more sensors 106, via one or more networks (e.g., radio technology). The communication interface 208 may include an electronic circuit specific to a standard that may enable wireless communication.

Referring to Figures 1 and 2, the one or more processors 202 may be configured to measure the one or more environmental parameters within the container at pre-determined time intervals. To measure the one or more environmental parameters, the one or more processors 202 may be configured to receive readings from the one or more sensors 106 and determine the values of the one or more environmental parameters based on the received readings.

In some embodiments, the pre-determined time intervals may be fixed time intervals. The one or more processors 202 may be configured to measure the one or more environmental parameters at fixed time intervals. In non-limiting examples, the pre-determined time intervals may include 5 minutes, 10 minutes, 15 minutes, and the like.

In some embodiments, the one or more processors 202 may be configured to detect activation of a purge mode with respect to the container 102. In some embodiments, the purge mode may be a CO₂ purge mode. In some embodiments, the purge mode may be an O₂ purge mode. In some embodiments, in the purge mode, the purging may be carried out within the container 102. In some embodiments, the purging can be with nitrogen (N2) and/or air. In some embodiments, in the CO₂ purge mode, the container 102 may be pressurized with nitrogen.

In some embodiments, the one or more processors 202 may be configured to initiate monitoring of the one or more environmental parameters in response to detecting activation of the purge mode. In some embodiments, the one or more processors 202 may be configured to measure the one or more environmental parameters till a de-activation of the purge mode. As an example, the values of CO₂ and O₂ within the container 102 may be measured at pre-determined time intervals (say, 15 minutes) upon activation of the CO₂ purge mode. As an example, the RQ parameter within the container 102 may be measured at pre-determined time intervals (say, 15 minutes) upon activation of the CO₂ purge mode. The measurements may continue till the CO₂ purge mode is de-activated.

In some embodiments, the one or more processors 202 may be configured to measure the one or more environmental parameters for a predefined period of time. That is, the one or more processors 202 may measure the one or more environmental parameters at pre-determined time intervals for the predefined period of time. As an example, the one or more processors 202 may measure the one or more environmental parameters at 15-minute intervals for a period of 2 hours.

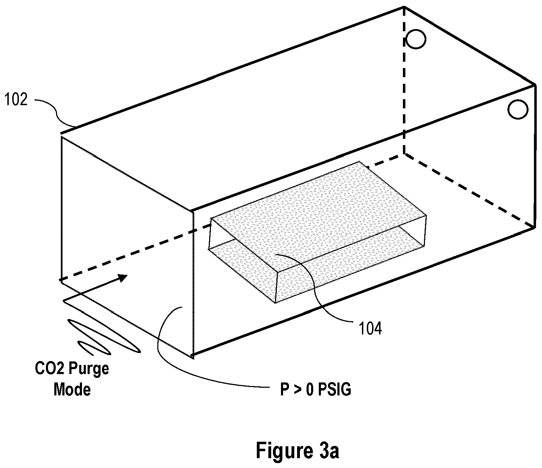

The one or more processors 202 may be configured to determine a respiration rate associated with each of the perishable items 104 based on the measured one or more environmental parameters. In some embodiments, the respiration rate may be determined in terms of CO₂ or O₂ units of ml(x)/kg-hr. In some embodiments, the one or more processors 202 may be configured to determine the respiration rate upon activation of the CO₂ purge mode. In some embodiments, the one or more processors 202 may be configured to measure the O₂ and CO₂ values within the container at several time instances (data points) over a period of time. In other embodiments, the RQ parameter may be measured by the one or more processors 202 at several time instances over the period of time. As described above, the RQ parameter within the container 102 may be measured upon activation of the CO₂ purge mode. In some embodiments, the container 102 may be associated with a positive pressure in the CO₂ purge mode. As shown in **Figure 3a****,** the determination of the respiration rate may be performed under specific pressure and mode condition. In particular, the determination of the respiration rate may be performed during the CO₂ purge mode and when the container 102 is associated with a positive pressure, i.e., when pressure within the container 102 may be greater than atmospheric pressure. In an embodiment, the positive pressure may refer to the condition when the pressure (P) within the container is greater than 0 PSIG (Pounds per Square Inch Gauge), thereby satisfying the equation P > 0 PSIG.

In some embodiments, the measured values at the several time instances may be considered upon meeting one or more pre-defined criteria. The one or more predefined criteria may include, for instance, a minimum of data points or a minimum threshold of coefficient of determination associated with the measurements. As would be understood to a skilled person, the coefficient of determination would be a parameter indicative of a prediction accuracy associated with the measurements and the prediction of respiration rate of the perishable items 104.

In some embodiments, one or more assumed parameters may be considered in order to determine the respirate rate. The one or more assumed parameters may include mass of cargo, free volume within the container 102, Nitrogen (N₂) flow, N₂ purity, and the like. In some embodiments, the one or more assumed parameters may be pre-stored in the memory 204.

The one or more processors 202 may be configured to determine, based on the determined respiration rate and the ripening database 204a, an initiation time for initiating ripening of the perishable items 104. The initiation time may indicate an optimal time for the ripening of the perishable items 104 to begin.

In some embodiments, the one or more processors 202 may be configured to receive one or more additional inputs indicative of a target time for the ripening of the perishable items 104. The one or more additional inputs may be based on a customer's request associated with the perishable items 104. The customer's request may relate to a ripening state of the perishable items 104 when the perishable items 104 have completed the transportation. For instance, the customer may desire to receive fully ripened perishable items 104 when the transportation of the perishable items 104 is complete. The target time may thus indicate when the desired ripening state of the perishable items 104 is to be achieved. In some embodiments, predicting ripening states of the perishable items may be carried out on the basis of a first trained mathematical model, such as a neural network (NN) model and/or a machine learning (ML) model. In some embodiments, the first trained mathematical model may be stored in the memory 204.

In some embodiments, the one or more processors 202 may be configured to determine the initiation time based on the received one or more additional inputs, i.e., based on the target time. For instance, in case the target time of achieving fully ripened perishable items 104 is 5 days, the initiation time may be determined based on the target of 5 days and the target ripening state of fully ripened.

The one or more processors 202 may be configured to trigger the ripening of the perishable items 104 at the initiation time. In some embodiments, to trigger the ripening of the perishable items 104, the one or more processors 202 may be configured to cause release of a ripening agent within the container 102. In some embodiments, the ripening agent may be ethylene.

In some embodiments, the one or more processors 202 may be configured to continue monitoring the one or more environmental parameters within the container 102 after triggering the ripening of the perishable items 104. Once the ripening is triggered, the characteristics of the perishable items 104 as well as the environmental parameters may change within the container 102. Accordingly, a dynamic adjustment of the environmental parameters may be required based on the ripening of the perishable items 104. As an example, in order to complete the ripening of the perishable items 104 at the target time, dynamic adjustment of the one or more environmental parameters may be required to avoid under-ripening or over-ripening of the perishable items 104. In some embodiments, dynamic adjustment of the one or more environmental parameters may be determined based on a second trained mathematical model, such as a neural network (NN) model and/or a machine learning (ML) model. In some embodiments, the second trained mathematical model may be stored in the memory 204.

The one or more processors 202 may thus be configured to adjust the one or more environmental parameters to complete the ripening of the perishable items at the target time. In some embodiments, adjusting the one or more environmental parameters causes a change in the respiration rate associated with each of the perishable items 104. This is because once the ripening is initiated, there is an inherent change in the characteristics of the perishable items 104, which causes a change in the respiration rates of the perishable items 104. Moreover, with a change in the respiration rates of the perishable items 104, the ripening may also be affected. In an example, an increased respiration rate may increase a rate of ripening of the perishable items 104 or cause the perishable items 104 to ripen at a faster pace.

In some embodiments, the one or more processors 202 may be configured to determine whether the respiration rate is greater than a pre-determined threshold. In some embodiments, the pre-determined threshold may be stored in the memory 204. Upon a determination that the respiration rate is greater than a pre-determined threshold, the one or more processors 202 may be configured to trigger an alarm associated with the container 102. The alarm may be indicative of an increase in the respiration rates of the perishable items 104 which may cause damage to the perishable items 104 within the container 102.

In some embodiments, the one or more processors 202 may be configured to store the measured one or more environmental parameters in the ripening database 204a. The ripening database 204a may be used for predicting initiation times for ripening of different batches of perishable items 104. In some embodiments, prediction of the initiation times may be carried out based on a third trained mathematical model, such as a neural network (NN) model and/or a machine learning (ML) model. In some embodiments, the third trained mathematical model may be stored in the memory 204.

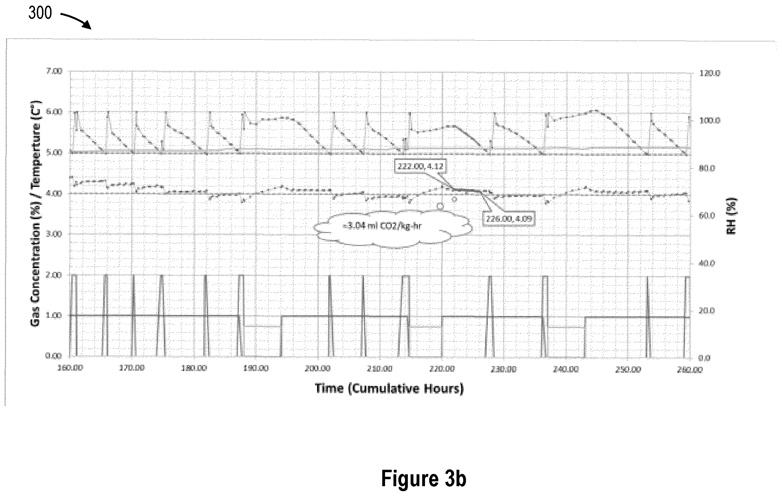

In some embodiments, the stored one or more environmental parameters may include the measured values of the one or more environmental parameters over the course of the transportation of the perishable items 104 from one place to another. Reference is made to **Figure 3b** which illustrates an exemplary graphical representation 300 of the measured values of the one or more environmental parameters over a period of time. In an embodiment, the period of time may be the time of the transportation of the perishable items 104 from one place to another. In an embodiment, the period of time may be any pre-determined period of time during the transportation of the perishable items 104 from one place to another.

As seen in Figure 3b, the values of gas concentration over the period of time are depicted. In an embodiment, the gas may be CO₂. In an embodiment, the gas may be O₂. The measurement of the gas concentration may be carried out at a particular temperature value within the container 102.

In some embodiments, the one or more processors 202 may be configured to display a ripening status of the perishable items 104 on a display unit. The ripening state may indicate a current ripening state of the perishable items 104. In some embodiments, the one or more processors 202 may be configured to display details related to the measured one or more environmental parameters on the display unit. In an embodiment, the display unit may be a display provided on the container 102. In an embodiment, the display unit may be a display associated with the system 110 and remote to the container 102.

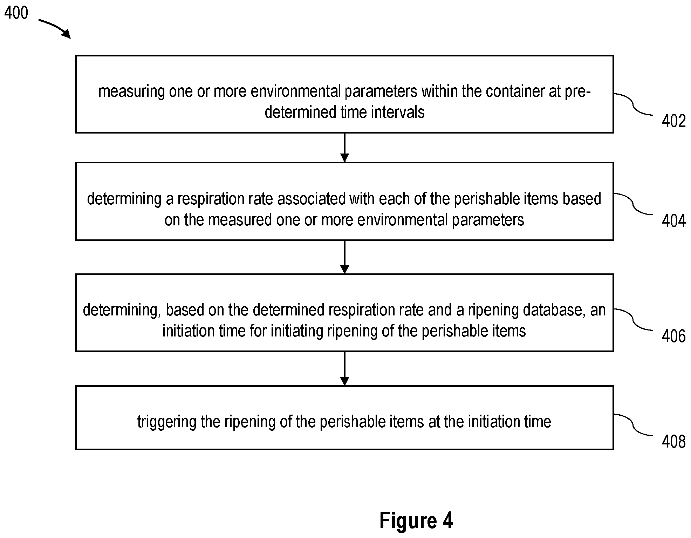

**Figure 4** illustrates a process flow depicting a method 400 for monitoring ripening of perishable items 104 stored within the container 102, in accordance with one or more embodiments of the invention. The method 400 may be described with reference to the container 102, and a skilled person will appreciate that the method may be performed for multiple different containers.

At step 402, the method 400 comprises measuring one or more environmental parameters within the container at pre-determined time intervals.

At step 404, the method 400 comprises determining the respiration rate associated with each of the perishable items 104 based on the measured one or more environmental parameters.

At step 406, the method 400 comprises determining, based on the determined respiration rate and a ripening database 204a, an initiation time for initiating ripening of the perishable items 104.

At step 408, the method 400 comprises triggering the ripening of the perishable items 104 at the initiation time.

While the above steps of Figure 4 are shown and described in a particular sequence, the steps may occur in variations to the sequence in accordance with various embodiments of the invention. Further, a detailed description related to the various steps of Figure 4 is already covered in the description related to Figures 1-2 and is omitted herein for the sake of brevity.

In some embodiments, the one or more processors 202 may be configured to execute instructions included in a computer program product. The computer program product may be embodied on a non-transitory computer readable medium. The computer program product may comprise instructions that, when executed by the one or more processors 202, cause the one or more processors 202 to perform the method steps are detailed with reference to Figure 4.

The invention provides methods and systems which allows effective monitoring of ripening and respiration rates of perishable items 104 which are to be transported from one location to another location. The conditions of the perishable items 104 may be indicated prior to completion of the transportation. Further, the on-board parameters may be adjusted in order to meet a customer's request for desired ripening of the perishable items 104.

As would be apparent to a person in the art, various working modifications may be made to the methods disclosed herein in order to implement the inventive concept as taught herein.

Moreover, the actions of any flow diagram need not be implemented in the order shown; nor do all of the acts necessarily need to be performed. Also, those acts that are not dependent on other acts may be performed in parallel with the other acts.

The drawings and the forgoing description give examples of embodiments. Those skilled in the art will appreciate that one or more of the described elements may well be combined into a single functional element. Alternatively, certain elements may be split into multiple functional elements. Elements from one embodiment may be added to another embodiment. For example, orders of processes described herein may be changed and are not limited to the manner described herein.

Benefits, other advantages, and solutions to problems have been described above with regard to specific embodiments. However, the benefits, advantages, solutions to problems, and any component(s) that may cause any benefit, advantage, or solution to occur or become more pronounced are not to be construed as a critical, required, or essential feature or component of any or all the claims.

While specific language has been used to describe the subject matter, any limitations arising on account thereto, are not intended. As would be apparent to a person in the art, various working modifications may be made to the method in order to implement the inventive concept as taught herein. The drawings and the foregoing description give examples of embodiments. Those skilled in the art will appreciate that one or more of the described elements may well be combined into a single functional element. Alternatively, certain elements may be split into multiple functional elements. Elements from one embodiment may be added to another embodiment.

## Claims

1. A method (400) for monitoring ripening of perishable items (104) stored within a container (102), the method (400) comprising:
measuring (402) one or more environmental parameters within the container (102) at pre-determined time intervals;
determining (404) a respiration rate associated with each of the perishable items (104) based on the measured one or more environmental parameters;
determining (406), based on the determined respiration rate and a ripening database (204a), an initiation time for initiating ripening of the perishable items (104); and
triggering (408) the ripening of the perishable items (104) at the initiation time.

2. The method (400) of claim 1, further comprising:
receiving one or more additional inputs indicative of a target time for the ripening of the perishable items (104); and
determining the initiation time based on the received one or more additional inputs.

3. The method (400) of claim 1 or 2, further comprising:
continue monitoring the one or more environmental parameters within the container (102) after triggering the ripening of the perishable items (104); and
adjusting the one or more environmental parameters to complete the ripening of the perishable items (104) at the target time, wherein adjusting the one or more environmental parameters causes a change in the respiration rate associated with each of the perishable items (104).

4. The method (400) of any preceding claim, wherein the one or more environmental parameters include CO₂, O₂, temperature, ethylene, and humidity.

5. The method (400) of any preceding claim, comprising storing the measured one or more environmental parameters in the ripening database (204a).

6. The method (400) of any preceding claim, comprising:
detecting activation of a purge mode with respect to the container (102); and
initiating monitoring of the one or more environmental parameters in response to detecting activation of the purge mode.

7. The method (400) of any preceding claim, comprising:
determining whether the respiration rate is greater than a pre-determined threshold; and
upon a determination that the respiration rate is greater than a pre-determined threshold, triggering an alarm associated with the container (102).

8. A system (110) to monitor ripening of perishable items (104) stored within a container (102), the system (110) comprising:
a memory (204)
one or more processors (202) communicatively coupled to the memory (204), the one or more processors (202) being configured to:
measure one or more environmental parameters within the container (102) at pre-determined time intervals;
determine a respiration rate associated with each of the perishable items (104) based on the measured one or more environmental parameters;
determine, based on the determined respiration rate and a ripening database (204a), an initiation time for initiating ripening of the perishable items (104); and
trigger the ripening of the perishable items (104) at the initiation time.

9. The system (110) of claim 8, wherein the one or more processors (202) are further configured to:
receive one or more additional inputs indicative of a target time for the ripening of the perishable items (104); and
determine the initiation time based on the received one or more additional inputs.

10. The system (110) of claim 8 or 9, wherein the one or more processors (202) are further configured to:
continuously monitor the one or more environmental parameters within the container after triggering the ripening of the perishable items (104); and
adjust the one or more environmental parameters to complete the ripening of the perishable items (104) at the target time, wherein adjusting the one or more environmental parameters causes a change in the respiration rate associated with each of the perishable items (104).

11. The system (110) of any of claims 8 to 10, wherein the one or more environmental parameters include CO₂, O₂, temperature, ethylene, and humidity.

12. The system (110) of any of claims 8 to 11, wherein the one or more processors (202) are further configured to store the measured one or more environmental parameters in the ripening database (204a).

13. The system (110) of any of claims 8 to 12, wherein the one or more processors (202) are configured to:
detect activation of a purge mode with respect to the container (102); and
initiate monitoring of the one or more environmental parameters in response to detecting activation of the purge mode.

14. The system (110) of any of claims 8 to 13, wherein the one or more processors (202) are configured to:
determine whether the respiration rate is greater than a pre-determined threshold; and
upon a determination that the respiration rate is greater than a pre-determined threshold, trigger an alarm associated with the container (102).
